Europäisches Patentamt

(19) European Patent Office

Office européen des brevets

(11) Numéro de publication: **0 068 057**
**B1**

(12) **FASCICULE DE BREVET EUROPEEN**

(45) Date de publication du fascicule du brevet:
27.12.85

(21) Numéro de dépôt: 81401696.0

(22) Date de dépôt: 27.10.81

(51) Int. Cl.⁴: **B 01 J 12/00**, B 01 J 19/24,
B 01 J 19/02, C 07 B 43/02,
C 07 C 79/04

(54) Réacteur de nitration d'hydrocarbures en phase gazeuse.

(30) Priorité: 26.06.81 US 278004

(43) Date de publication de la demande:
05.01.83 Bulletin 83/1

(45) Mention de la délivrance du brevet:
27.12.85 Bulletin 85/52

(84) Etats contractants désignés:
AT BE CH DE FR GB IT LI LU NL SE

(56) Documents cités:
EP - A - 0 017 559
DE - A - 1 811 596
DE - A - 2 815 560
FR - A - 1 213 939

(73) Titulaire: SOCIETE CHIMIQUE DE LA GRANDE
PAROISSE AZOTE ET PRODUITS CHIMIQUES, 8, rue
Cognacq-Jay, F-75321 Paris Cedex 07 (FR)

(72) Inventeur: Lhonore, Pierre, 1090, bd Jeanne d'Arc,
F-59500 Douai (FR)
Inventeur: Quibel, Jacques, 40, rue de la Muette,
F-78600 Maisons-Laffitte (FR)
Inventeur: Jacquinot, Bernard, 59, rue Léon Bathiat,
F-59500 Douai (FR)
Inventeur: Jestin, Yvon, 99, rue Georges Sand,
F-92500 Rueil-Malmaison (FR)
Inventeur: Pelletier, Robert, 113, rue du Faubourg
Poissonnière, F-75009 Paris (FR)

(74) Mandataire: Bouton Neuvy, Liliane et al, L'Air liquide,
Société Anonyme pour L'Etude et L'Exploitation des
Procédés Georges Claude 75, Quai d'Orsay,
F-75321 Paris Cedex 07 (FR)

**Description**

La présente invention concerne un type de réacteur utilisable dans toutes les nitrations d'hydrocarbures en phase gazeuse sous pression. Ce réacteur par sa structure permet l'obtention d'un régime thermique à progression régulière au cours de la réaction de nitration, c'est-à-dire que l'évolution de la courbe de température de la réaction est continue et progressive, sans accélération importante de la vitesse réactionnelle et sans montée brusque de la température en divers endroits de la zone réactionnelle. Ce réacteur est spécialement conçu pour avoir un bon échange de chaleur et une maîtrise de la courbe d'élèvation de la température pendant la réaction.

Il a, d'autre part, été trouvé une qualité de métal, pour la réalisation d'un tel réacteur de l'ensemble mélangeur-répartiteur, susceptible de permettre des nitrations satisfaisantes, de manière telle que les réactions concurrentes d'oxydation demeurent peu importantes au regard des réactions de nitration. Ce métal est, d'autre part, capable de résister à la pression dans les tubes pouvant atteindre 100 bars à la température de fonctionnement.

Le réacteur est constitué par un faisceau de tubes dans lesquels le mélange est le plus régulièrement réparti.

La qualité de la réaction de nitration conduite à partir d'un mélange de composition appropriée (hydrocarbure, agent nitrant oxygéné et éventuellement gaz inertes, et composés organiques tels nitroparaffines recyclées) dépend: de la qualité des échanges thermiques du réacteur; de l'homogénéité du mélange réactionnel et de l'uniformité de sa répartition entre les divers tubes du faisceau du réacteur, et de la nature du métal qui constitue le faisceau du réacteur ainsi que l'ensemble mélangeur-répartiteur.

Dans le réacteur de nitration d'hydrocarbures en phase gazeuse sous pression, constitué par une enceinte réactionnelle à l'intérieur de laquelle un faisceau tubulaire ou platulaire est en contact avec un fluide caloporteur de forte capacité d'échange thermique, les échanges thermiques sont assurés par une circulation à co-courant ou contre-courant, de préférence à co-courant, d'un fluide caloporteur capable de fonctionner de manière satisfaisante aux températures de nitration, par exemple entre 200 et 450°C. La circulation du fluide caloporteur est réalisée de manière à assurer à la fois un échange thermique suffisant au niveau de la zone où la réaction est maximale et un préchauffage — des réactifs jusqu'à la température de début de réaction-suffisamment progressif pour éviter une réaction trop vive.

Le fluide caloporteur et son mode de circulation à l'extérieur du faisceau sont choisis de telle manière que la température de la peau extérieure des tubes soit maintenue constante à 25°C, puis entre le point le plus froid et le point le plus chaud.

Le dimensionnement des tubes du faisceau joue un rôle important quant à la croissance régulière de la température des gaz au cours de la réaction. Un choix approprié de ces dimensions permet de contrôler le profil de température à l'intérieur des tubes tant dans la zone de préchauffage que dans la zone de réaction et que dans la partie correspondant au refroidissement des produits de la réaction. En particulier, une augmentation progressive de la température dans la zone de préchauffage assure un régime thermique stable et évite l'emballement de la réaction qui se traduirait par une température maximale trop élevée pour une nitration correcte.

Le choix judicieux du rapport entre la surface du faisceau tubulaire en contact avec le milieu réactionnel et le volume de l'enceinte réactionnelle, S/V, a une influence favorable sur la croissance régulière de la température au cours de la réaction, quand ce rapport est compris entre 22 et 425 m$^{-1}$, de préférence 50 et 250 m$^{-1}$ et en particulier 120 et 250 m$^{-1}$, on obtient un bon écoulement des gaz réactionnels et un bon échange thermique.

La forme et la section des tubes est indifférente (circulaire, ovale, ou platulaire) dès lors qu'elle permet le bon écoulement du fluide gazeux et du fluide caloporteur, assurant le transfert requis. Pour cela, le périmètre intérieur des tubes du faisceau doit rester inférieur à 800 millimètres, de préférence inférieur à 500 millimètres, tandis que l'épaisseur de la veine gazeuse ne doit pas excéder 150 millimètres, de préférence 50 millimètres.

La longueur des tubes doit être suffisante pour que la réaction se produisant, la température du mélange gazeux soit ramenée à une valeur proche de la température du fluide caloporteur, constitué par exemple de sels fondus. La longueur minimale dépend de la température d'entrée dans les tubes; si celle-ci est faible, la partie des tubes consacrée au préchauffage des réactifs, jusqu'à la température où la réaction commence est plus longue, donc la longueur totale nécessaire est plus importante. Des longueurs comprises entre 8 et 12 mètres conviennent parfaitement.

Les valeurs des rapports Q/US et Q'/US doivent être convenablement choisies pour que la réaction puisse avoir lieu dans de bonnes conditions. Q désigne la chaleur dégagée par la réaction, en considérant que celle-ci commence, par exemple, à partir de 260°C, Q' désigne la chaleur totale échangée avec le fluide caloporteur, c'est-à-dire la somme de chaleur cédée au mélange des gaz réactionnels pour porter leur température à 260°C et de la chaleur cédée au fluide caloporteur au cours de la réaction et du refroidissement des gaz. U désigne le coefficient global d'échange thermique théorique calculé pour les gaz réactionnels à la température du fluide caloporteur et se rapportant à la surface externe de l'enceinte réactionnelle, et S la surface d'échange interne disponible rapportée à

2

un tube de longueur 12 mètres. Ces rapports Q/SU et Q'/SU ont la dimension d'une température, et sont valables quel que soit le dimensionnement des tubes. Les rapports Q/SU compris entre 20 et 120°C, et de préférence entre 40 et 90°C, tandis que les rapports Q'/SU sont compris entre 25 et 160°C, de préférence 50 et 130°C, permettent la réalisation d'une réaction de nitration dans d'exellentes conditions. Le coefficient global d'échange U peut avantageusement être compris entre 20 et 300 Kcal/hm²°C, et de préférence entre 30 et 200.

Les meilleurs échanges thermiques sont obtenus, d'une part grâce au choix d'un fluide caloporteur approprié, choisi parmi les mélanges de sels fondus, tels nitrites et nitrates de métaux alcalins, et d'autre part grâce à une circulation efficace du fluide caloporteur, caractérisée par une réduction maximale des zones de moindre qualité de transfert thermique. Un excellent transfert thermique est obtenu par une circulation transversale du fluide caloporteur vis-à-vis du faisceau.

Le faisceau tubulaire ou platulaire est équipé de chicanes du type segmenté, disque ou couronne, réparties irrégulièrement touts au long de l'axe du faisceau; les distances séparant deux chicanes étant minimales au droit de la zone où se produit la réaction de nitration. Cette disposition a pour but d'assurer les meilleurs échanges thermiques entre le fluide caloporteur et les gaz réactionnels. Elle est destinée à réduire le plus possible les zones de moindre transfert thermique dans la zone où la réaction se produit et dans la zone où les effluents sont refroidis.

De plus, la distribution des gaz réactionnels à l'entrée du faisceau tubulaire, la conception de celui-ci ainsi que la circulation du fluide caloporteur sont telles que de préférence le réacteur aura la plus grande symétrie possible autour d'une axe parallèle aux tubes. Le faisceau comporte ou non des tubes dans la partie centrale.

Le réacteur est alimenté par un ensemble mélangeur-répartiteur destiné d'une part, à assurer un mélange homogène de l'ensemble des réactifs et, d'autre part, à répartir ce mélange homogène de façon régulière entre les divers tubes.

Le mélangeur assure un mélange homogène de l'ensemble des fluides réactionnels tel que les écarts maximaux de concentration en tout point du répartiteur n'excède pas 3%, de préférence 1%.

L'obtention de produits de la nitration avec de bons rendements dépend également du maintien régulier des débits, donc des temps de séjour dans les divers tubes du faisceau, ceux-ci devant être chargés de manière aussi identique que possible. Le mélangeur-répartiteur est équipé, à cet effet, d'un dispositif statique approprié, qui répartit régulièrement le débit dans les tubes du faisceau de manière telle que la différence maximale de charge entre le tube le plus chargé et le tube le moins chargé n'excède pas 3%.

Le mélange répartiteur peut être conçu avec ou sans circulation du fluide caloporteur sur tout ou partie des zones de mélange et de distribution, le fluide caloporteur ne devant jamais être en contact direct avec les gaz.

Le mélange et la distribution doivent intervenir dans un temps suffisamment court, au plus 0,5 seconde, et de préférence inférieure à 0,1 seconde. Les vitesses des gaz dans cet appareil mélangeur-répartiteur restent avantageusement comprises entre 3 et 50 mètres par seconde.

Le mélangeur-répartiteur entre les différents tubes du faisceau, combinant la technologie de mélange dynamique et statique, est muni d'une succession d'élargissements et de rétrécissements des sections de passage des gaz, en particulier d'un rétrécissement important de la section de passage au droit de chaque tube du faisceau; cette restriction permettant une meilleure répartition des gaz entre chaque tube.

Le faisceau tubulaire ainsi que l'ensemble mélangeur-repartiteur doivent être réalisés en métaux capables de résister aux corrosions provoquées par l'agent nitrant et au fluide caloporteur. Ce métal doit, en outre, être choisi de telle sorte qu'il n'ait pas pour effet de favoriser les réactions d'oxydation qui concurrencent la nitration. En particulier, certains aciers inoxydables austéniques au nickel-chrome donnent toute satisfaction. Ils sont du type Z 12 CNS 25-20; Z 3 CN 18-10, AISI 310 S et dans les Inconel, l'Inconel 600 ou ZNC Se 72-14 (norme AFNOR).

Composition des aciers précités:

    Z 3 CN 18-10 C, max. 0,035
        Cr 18,5    Ni 10,5
    Z 12 CNS 25-20 C, max. 0,10
        Cr 25    Ni 20
    et A 310 S.

    Inconel 600 C, max. 0,2
        Cr 14-17    Ni ≤72    Fe 6-10    Cu <0,7    Si <0,5    Mn <1

Le réacteur de l'invention est caractérisé par les dispositions structurelles qui seront précisées ci-après et les paramètres relatifs à la température de peau des tubes, à leur diamètre, aux rapports S/V, Q/S, Q/US et U.

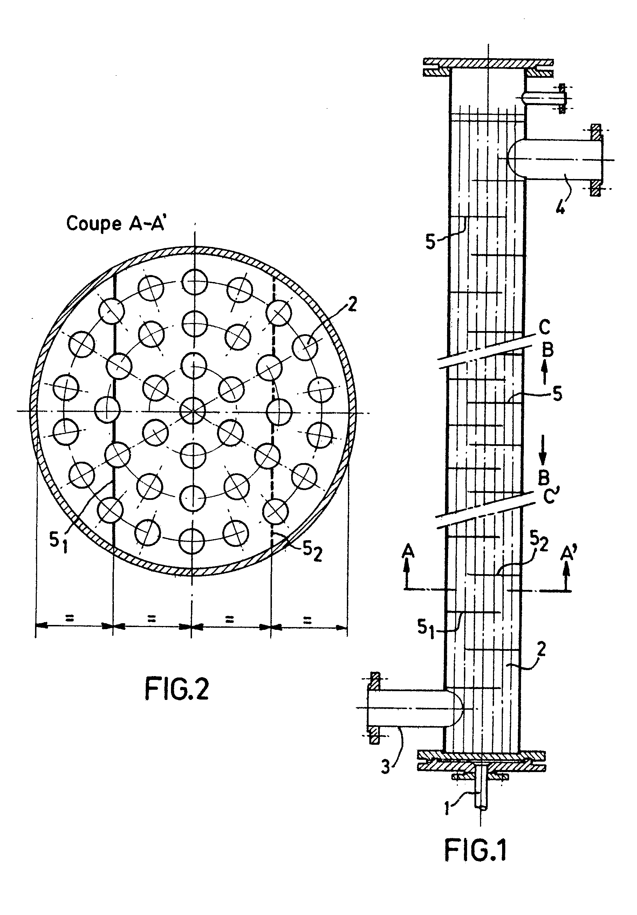

La figure 1 correspond à la coupe verticale du réacteur (les parties pleines étant représentées en grisailles).

Le mélange réactionnel est introduit par la partie inférieure du faisceau tubulaire vertical (1), les lignes verticales (2) représentent quelques tubes dans lesquels le mélange réactionnel circule de bas en haut.

Le fluide caloporteur circule dans le même sens, à co-courant des gaz. Il est introduit et extrait par les dispositifs (3) et (4). La circulation du fluide caloporteur est contrôlée par la disposition des chicanes du type segmenté (5), réparties irrégulièrement et avec une distance minimale au droit de la zone réactionnelle, c'est-à-dire dans la partie centrale du tube entre B et B'. (Les intervalles pointillés C et C' correspondent aux parties non représentées du tube dans la hauteur des 12 mètres).

La figure 2 correspond à une coupe transversale avec répartition des 37 tubes (2) au niveau AA' de la figure 1. On remarque les chicanes segmentées $5_1$ et $5_2$.

Les figures 3, 4 et 5 représentent la distribution des gaz. La figure 3 représente la partie inférieure du faisceau tubulaire. La figure 4 correspond à la coupe transversale AA' de l'ensemble des tubes du faisceau et à la répartition du mélange réactionnel dans les tubes. La figure 5 correspond à la coupe transversale BB' de distribution du mélange réactionnel.

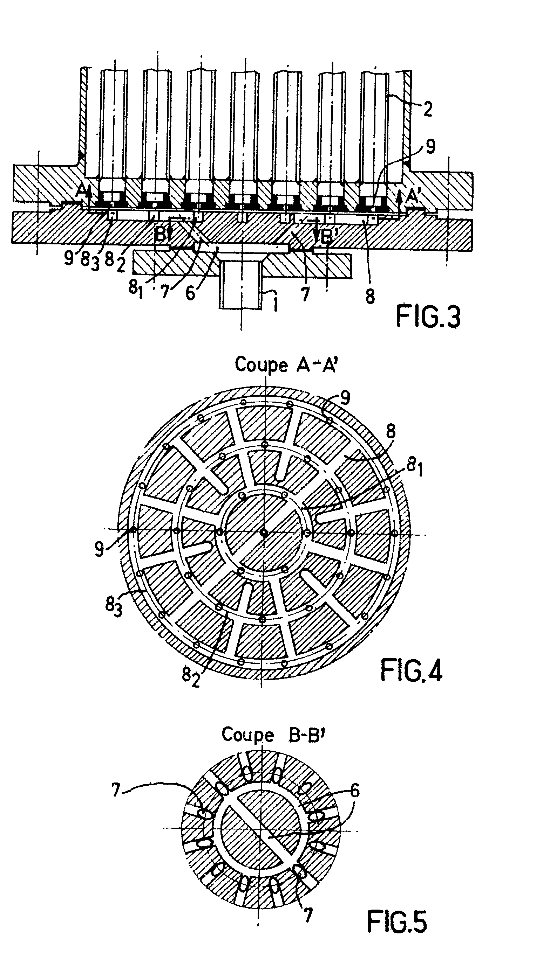

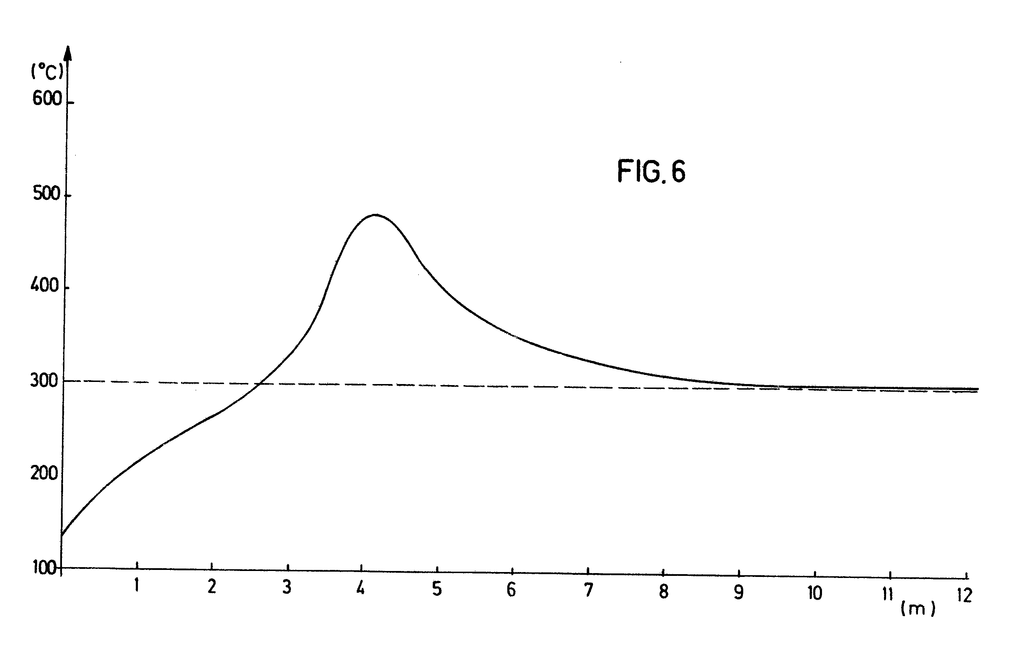

Le mélangeur des gaz réactionnels n'est pas représenté sur la figure 3. A la sortie du mélangeur, les gaz arrivent par la conduite (1) à la base du dispositif répartiteur-distributeur qui provoque des accélérations et décélérations dans la zone de distribution, en (6) (fig. 3 et 5). Les gaz entrent dans une zone de décélération à partir de laquelle rayonnent des conduits (7) dans lesquels la vitesse des gaz est accélérée. A ce niveau, les gaz distribués par les conduits (7) sont répartis selon la représentation de la Figure 4, selon la coupe AA' par les dispositifs répartiteurs (8), représentés sur la Figure 4 par les trois couronnes distributrices ($8_1$, $8_2$, $8_3$); dans cette zone, il y a décélération. Les dispositifs répartiteurs sont à la base des tubes et dans l'axe de ceux-ci, puis les gaz sont contraints vers un passage calibré (9) à l'entrée et dans l'axe des tubes, qui entraine une accélération finale des gaz à l'entrée des tubes.

Il est donné des exemples, qui illustrent l'invention à titre non limitatif.

## Exemple 1

Réacteur de nitration d'hydrocarbures en phase gazeuse sous pression constitué par un faisceau de 37 tubes.

Le faisceau tubulaire est formé de 37 tubes d'acier inoxydable Z 12 CNS 25-20 (C 0, 10, Cr 25, Ni 20) de longueur 12 mètres, de diamètre intérieur et extérieur de 21,7 et 26,9 millimètres répartis selon un pas triangulaire de 46 millimètres. On a la possibilité d'obturer un certain nombre de tubes pour utiliser n tubes du faisceau, n pouvant être choisi entre 30 et 37 selon les conditions de fonctionnement recherchées.

Le rapport entre la surface interne des tubes et le volume intérieur du faisceau est de 184 $m^{-1}$.

Les échanges thermiques sont assurés par une circulation à cocourant du fluide caloporteur constitué par un mélange de sels fondus ($NaNO_2$), 7% de nitrate de sodium ($NaNO_3$) et 53% de nitrate de potassium ($KNO_3$).

A l'intérieur des tubes les chicanes segmentées sont disposées irrégulièrement de manière à permettre une variation dans un rapport de 1 à 3 du fluide caloporteur.

Les rapports Q/SU et Q'/SU peuvent varier de 49 à 75 pour Q/SU et de 60 à 120 pour Q'/SU, tandis que le rapport entre la chaleur à évacuer peut varier entre 3000 et 6500 Kcal/$hm^2$.

Les gaz réactionnels sont mélangés dans un mélangeur double tube muni d'aillettes hélicoïdales assurant un mélange homogène de qualité telle que les écarts maximaux de concentration en tout point du répartiteur n'excède pas 3%, et ce dans un délai de 0,03 à 0,04 secondes pour des vitesses de 4 à 25 mètres par seconde.

Le mélange des gaz réactionnels circule ensuite dans un distributeur-répartiteur où il est soumis à une succession de décélérations et d'accélérations au cours desquelles les vitesses sont maintenues dans un intervalle de 2 à 25 mètres par seconde. L'admission du mélange réactionnel dans chaque tube du faisceau se fait à travers un orifice calibré qui provoque une perte de charge importante et des vitesses des gaz allant de 15 à 30 mètres/seconde, qui permet une bonne répartition du mélange gazeux entre les divers tubes du faisceau: l'écart maximal entre les débits à l'entrée de chaque tube n'excède pas 3%.

Par l'intermédiaire d'un dispositif de thermocouples fixes, ainsi que par celui d'un thermocouple mobile, on connait le profil thermique des températures de l'enceinte réactionnelle; cette connaissance permettant de faire varier les paramètres de la réaction, tels Q/SU, U, temps de contact, débit du fluide caloporteur, tout en contrôlant l'évolution de la nitration.

1a. Nitration dans ce réacteur ayant 36 tubes en service

Températures des sels fondus: entrée = 299° C; sortie = 300° C
Pression des gaz dans le mélangeur-répartiteur: 8.8 Kgs/$cm^2$ effectifs

Tableau I

| | entrée reacteur kg/h | sortie reacteur kg/h |
|---|---|---|
| $C_3H_8$ | 380.78 | 335.79 |
| $C_2H_6$ | 1.74 | 1.73 |
| $C_4H_{10}$ | 3.76 | 2.76 |
| $NO_2$ | 102.08 | |
| $O_2$ | 20.64 | |
| $N_2$ | 71.16 | 72. |
| CO | | 9.24 |
| $CO_2$ | | 15.41 |
| NO | | 38.78 |
| $H_2O$ | | 32.05 |
| méthanol $CH_3OH$ | | 0.05 |
| acétaldéhyde $CH_3CHO$ | | 2.97 |
| éthanol $C_2H_5OH$ | | — |
| acétonitrile $CH_3CN$ | | 1.86 |
| acétone $(CH_3)_2CO$ | | 4.24 |
| Propionitrile $C_2H_5CN$ | | .56 |
| Nitrométhane $CH_3NO_2$ | | 21.43 |
| Nitroéthane $C_2H_5NO_2$ | | 5.32 |
| 2 Nitro-propane $(CH_3)_2CHNO_2$ | | 25.12 |
| 1 Nitro-propane $C_3H_7NO_2$ | | 10.57 |
| Acide nitreux $HNO_2$ | | .98 |
| Acide nitrique $HNO_3$ | | .51 |

Temps de séjour calculé comme suit: 8,5 secondes, temps de séjour pour un débit gazeux constant égal au débit volumique à l'entrée des tubes, sous une pression constante égale à la pression d'entrée et à une température constante égale à la température moyenne du fluide caloporteur.

Q = chaleur dégagée au cours de la réaction (à partir de 260°C) = 120 172 Kcal/h
chaleur fournie aux réactants pour les porter à 260°C = 31 972 Kcal/h
Q' = chaleur totale échangée entre les gaz et le fluide caloporteur = 152 144 Kcal/h

Q/S = 4005 Kcal/hm2     Q'/S = 5.166 Kcal/hm2
Q/US = 63°C     Q'/US = 80°C
U = 64,6 Kcal/hm2C

Le profil de température dans les tubes du réacteur apparait selon la courbe de la figure 6, la distance parcourue dans le tube par le mélange réactionnel exprimée en mètres est portée en abscisse (m) et les températures en °C en ordonnée.

1b. Nitration dans réacteur de 36 tubes

Températures des sels fondus: entrée = 329°C; sortie = 331°C
Pression entrée tubes: 9,7 Kg/cm$^2$ effectifs

Tableau  II

|  | entrée, kg/h | sortie, kg/h |
|---|---|---|
| $C_3H_8$ | 417.21 | 377.67 |
| $C_2H_6$ | 1.43 | 1.42 |
| $C_4H_{10}$ | 4.49 | 2.56 |
| $NO_2$ | 121.99 | |
| $O_2$ | 28.98 | |
| $N_2$ | 96.88 | 95.47 |
| CO | | 8.92 |
| $CO_2$ | .51 | 17.35 |
| NO | | 68.32 |
| $H_2O$ | | 42.68 |
| $CH_3OH$ | | .51 |
| $CH_3CHO$ | | 11.65 |
| $C_2H_5OH$ | | — |
| $CH_3CN$ | | .03 |
| $(CH_3)_2CO$ | | 3.10 |
| $C_2H_5CN$ | | — |
| $CH_3NO_2$ | .11 | 18.84 |
| $C_2H_5NO_2$ | 7.92 | 11.92 |
| $(CH_3)_2CHNO_2$ | 46.82 | 55.80 |
| $C_3H_7NO_2$ | 3.56 | 13.04 |
| $HNO_2$ | | .42 |
| $HNO_3$ | | 2.96 |

Temps de séjour selon définition exemple I = 6.5 secondes

Q     = 132 950 Kcal/h       Q'     = 174 850 Kcal/h
Q/US = 49                    Q'/US = 64,5
Q/S  = 4514 Kcal/hm2         Q'/S  = 5940 Kcal/hm2
U     = 92 Kcal/hm2°C

**0 068 057**

## Exemple II

Réacteur de nitration d'hydrocarbures en phase gazeuse sous pression constitué par un faisceau de 1156 tubes.

Le faisceau tubulaire de ce réacteur est constitué de 1156 tubes en acier inoxydable du type Z 12 CNS 25-20 (C 0,10, Cr 25, Ni 20 silicium ≤1) ou Al Si 310, de diamètres intérieur et extérieur de 22,10 et 25,4 millimètres et de longueur 12 mètres. Le rapport entre la surface interne des tubes du faisceau et le volume intérieur du faisceau est de 181 $m^{-1}$.

Pour une nitration de mélange éthane-propane, les rapports Q/SU et Q'/SU peuvent varier entre 55 et 80°C pour Q/SU, et entre 70 et 105°C pour Q'/SU.

Comme dans le réacteur précédent, les échanges thermiques sont assurés par une circulation à co-courant d'un mélange de sels fondus à 40% en masse de $NaNO_2$, 7% de $NaNO_3$ et 53% de $KNO_3$.

Le faisceau tubulaire présente une symétrie de révolution par rapport à un axe parallèle aux tubes, les chicanes disposées à l'intérieur du faisceau tubulaire sont du type disques et couronnes, et sont réparties de façon irrégulière le long de l'axe du faisceau, les distances séparant deux chicanes étant minimales au droit de la zône où se produit la réaction.

Le mélange des gaz réactionnels, la distribution-répartition du mélange, son admission, le contrôle thermique des températures de l'enceinte réactionnelle sont réalisés dans les mêmes conditions que dans l'exemple 1.

Ce type de réacteur est adapté à l'échelle industrielle à la nitration de tout hydrocarbure ou mélange d'hydrocarbures saturés inférieurs à $C_5$, et par exemple à la nitration d'un mélange d'éthane et de propane, selon le bilan suivant:

Pression d'entrée = 11 bars effectifs;
température des sels fondus = 330°C;
Température d'entrée des gaz = 150°C/Température de sortie = 340°C.

Tableau III

| | entrée tubes, kg/h | sortie tubes, kg/h |
|---|---|---|
| $C_2H_6$ | 6554.79 | 6176.43 |
| $C_3H_8$ | 6569.38 | 5733.33 |
| $C_4H_{10}$ | 1.06 | .96 |
| iso $C_4H_{10}$ | 5.01 | 4.00 |
| CO | 1813.41 | 2176.13 |
| $CO_2$ | 3864.15 | 4672.09 |
| $N_2$ | 41.35 | 41.35 |
| $O_2$ | 734.44 | |
| NO | 38.66 | 1985.47 |
| $NO_2$ | 3958.94 | |
| $N_2O_4$ | | |
| $N_2O$ | 67.86 | 67.86 |
| $H_2O$ | 72.35 | 991.36 |
| Nitrométhane | .24 | 585.71 |
| Nitroéthane | 55.77 | 475.16 |
| 1 Nitro-Propane | 149.98 | 352.07 |
| 2 Nitro-Propane | 874.79 | 929.95 |

7

Tableau III (Suit)

| | entrée tubes, kg/h | sortie tubes, kg/h |
|---|---|---|
| 2 Nitro-Butane | .08 | 1.14 |
| Méthanol | | 52.12 |
| Ethanol | | 15.20 |
| Formaldéhyde | | 12.31 |
| Acétaldéhyde | | 242.06 |
| Acétone | | 106.38 |
| Acide Formique | | .46 |
| Acide Acétique | | 6.08 |
| Acétonitrile | | 30.39 |
| Propionitrile | | 1.98 |
| $C\,HNO_2$ | | 29.33 |
| $HNO_3$ | | 106.37 |

## Revendications

1. Réacteur de nitration en phase gazeuse sous pression comprenant une enceinte réactionnelle contenant un faisceau tubulaire ou platulaire vertical (2), une conduite d'introduction des gaz réactionnels (1) à la partie inférieure du réacteur, des dispositifs (3) et (4) d'introduction et d'extraction du fluide caloporteur, caractérisé en ce que le faisceau tubulaire ou platulaire est équipé de cloisonnements segmentés du type chicane, disque ou couronne, répartis irrégulièrement tout au long de l'axe du faisceau; les distances séparant deux cloisonnements segmentés étant minimales dans la partie centrale du faisceau au droit de la zone réactionnelle.

2. Réacteur de nitration en phase gazeuse sous pression selon la revendication 1, caractérisé en ce que le réacteur est équipé à la base d'un dispositif répartiteur — distributeur entre les différents tubes du faisceau, muni d'une succession d'élargissements et de rétrécissements de passage des gaz, en particulier d'un rétrécissement important de la section de passage au droit de chaque tube du faisceau.

3. Réacteur de nitration en phase gazeuse sous pression selon la revendication 2, caractérisé en ce que le répartiteur — distributeur comprend la zone de distribution des gaz (6) à partir de laquelle rayonnent les conduits (7) aboutissant aux dispositifs répartiteurs (8) en communication avec des couronnes distributrices ($8_1$, $8_2$, $8_3$), les dits dispositifs répartiteurs étant dans l'axe des tubes du faisceau (2) et à la base de ceux-ci, eux-mêmes munis à leur entrée et dans l'axe d'un passage calibré (9).

4. Réacteur de nitration en phase gazeuse sous pression selon la revendication 1, comprenant une enceinte réactionnelle contenant un faisceau tubulaire ou platulaire vertical, dont le périmètre intérieur des tubes est inférieur à 800 millimètres, caractérisé en ce que le rapport entre la surface du faisceau en contact avec le milieu réactionnel dite surface d'échange disponible, et le volume de l'enceinte réactionnelle S/V est compris entre 22 et 425 m$^{-1}$, de préférence entre 50 et 250 m$^{-1}$ et en particulier 120 et 250 m$^{-1}$, l'épaisseur de la veine gazeuse n'excédant pas 150 millimètres.

5. Réacteur de nitration en phase gazeuse sous pression comprenant un faisceau tubulaire selon la revendication 1, et un fluide caloporteur de nature appropriée, caractérisé en ce que la répartition des cloisonnements segmentés est telle que la température de la peau extérieure des tubes soit maintenue constante à 25° C près entre le point le plus chaud et le point le plus froid et en outre les échanges thermiques étant tels que les paramètres-Q désignant la chaleur dégagée par la réaction, S la surface d'échange disponible, U le coefficient global d'échange thermique et Q' la chaleur totale échangée avec le fluide caloporteur-Q/US soit compris entre 30 et 130, Q'/US entre 30 et 180 et U entre 12 et 200.

6. Réacteur de nitration en phase gazeuse sous pression selon la revendication 2, caractérisé en ce que le temps de séjour du mélange gazeux réactionnel dans le dispositif répartiteur-distributeur n'exède pas 0,5 seconde, de préférence 0,1 seconde, les vitesses du gaz dans l'ensemble du dispositif étant comprises entre 3 et 50 mètres/seconde, la répartition du débit dans les tubes du faisceau étant telle que la différence maximale de charge entre le tube le plus chargé et le tube le moins chargé soit au plus égale à 3%.

7. Réacteur de nitration selon la revendication 1, dont le faisceau et l'ensemble répartiteur-distributeur sont constitués par des métaux résistant à la corrosion, caractérisé en ce qu'ils sont choisis parmi les aciers inoxydables austénitiques au nickel chrome.

## Patentansprüche

1. Reaktor zur Nitrierung in der Gasphase unter Druck mit einem ein vertikales Röhren- oder Plattenbündel (2) enthaltenden Reaktionsraum, einer Einführleitung für Reaktionsgase (1) im unteren Teil des Reaktors, Einrichtungen (3) und (4) zur Einführung und Abführung von Wärmeaustauschfluid, dadurch gekennzeichnet, daß das Röhren- oder Plattenbündel mit in Segmente geteilten Unterteilungen vom Ablenkplatten-, Scheiben- oder Kranztyp, die unregelmäßig längs der Bündelachse verteilt sind, ausgestattet ist, wobei die zwei in Segmente geteilte Unterteilungen trennenden Abstände im Mittelteil des Bündels längs der Reaktionszone am kleinsten sind.

2. Reaktor zur Nitrierung in der Gasphase unter Druck nach Anspruch 1, dadurch gekennzeichnet, daß der Reaktor am Fuß mit einer Verteiler-Aufteilereinrichtung unter den verschiedenen Röhren des Bündels ausgerüstet ist, die mit einer Folge von Erweiterungen und Verengungen des Gasdurchgangs, insbesondere einer großen Erweiterung des Durchgangsabschnittes längs einer jeden Röhre des Bündels, versehen ist.

3. Reaktor zur Nitrierung in der Gasphase unter Druck nach Anspruch 2, dadurch gekennzeichnet, daß die Verteiler-Aufteilereinrichtung die Gasverteilungszone (6) aufweist, von der aus die Leitungen (7) ausgehen, welche in Verteilereinrichtungen (8) in Verbindung mit den Verteilerkränzen ($8_1$, $8_2$, $8_3$) münden, wobei diese Verteilereinrichtungen in der Achse der Röhren des Bündels (2) und am Fuß dieser liegen und dieselben an ihrem Eintritt und in der Achse mit einem kalibrierten Durchgang (9) versehen sind.

4. Reaktor zur Nitrierung in der Gasphase unter Druck nach Anspruch 1 mit einem Reaktionsraum, der ein vertikales Röhren- oder Plattenbündel enthält, dessen Innenumfang der Röhren kleiner als 800 mm ist, dadurch gekennzeichnet, daß das Verhältnis zwischen der Oberfläche des Bündels in Kontakt mit dem Reaktionsmedium, genannt verfügbare Austauschfläche, und dem Volumen des Reaktionsraumes, S/V, zwischen 22 und 425 m$^{-1}$, vorzugsweise zwischen 50 und 250 m$^{-1}$ und besonders zwischen 120 und 250 m$^{-1}$ liegt und die Dicke der Gasader 150 mm nicht überschreitet.

5. Reaktor zur Nitrierung in der Gasphase unter Druck mit einem Röhrenbündel nach Anspruch 1 und einem Wärmeaustauschfluid von geeigneter Natur, dadurch gekennzeichnet, daß die Aufteilung der in Segmente geteilten Unterteilungen derart ist, daß die Temperatur der Außenhaut der Röhren konstant nahe 25°C zwischen dem wärmsten Punkt und dem kältesten Punkt gehalten wird und daß außerdem der Wärmeaustausch derart ist, daß die Parameter, wobei Q die durch die Reaktion abgegebene Wärme, S die verfügbare Austauschfläche, U den Gesamtwärmeaustauschkoeffizienten und Q' die gesamte mit dem Wärmeaustauschfluid ausgetauschte Wärme bedeutet, Q/US zwischen 30 und 130, Q'/US zwischen 30 und 180 und U zwischen 12 und 200 liegen.

6. Reaktor zur Nitrierung in der Gasphase unter Druck nach Anspruch 2, dadurch gekennzeichnet, daß die Verweilzeit des Reaktionsgasgemisches in der Verteiler-Aufteilereinrichtung 0,5 sec, vorzugsweise 0,1 sec nicht überschreitet und die Aufteilung des Durchsatzes in den Röhren des Bündels derart ist, daß der Maximalunterschied der Beladung zwischen der am meisten beladenen Röhre und der am wenigsten beladenen Röhre höchstens 3% ist.

7. Reaktor zur Nitrierung nach Anspruch 1, dessen Bündel und Verteiler-Aufteilereinrichtung aus korrosionsbeständigen Metallen bestehen, dadurch gekennzeichnet, daß sie unter den rostfreien austenitischen Nickel-Chrom-Stählen ausgewählt sind.

## Claims

1. Reactor for the nitration in the gaseous phase under pressure comprising a reaction space containing a vertical tubular of plate like bundle (2), a conduit for the introduction of the reaction gases (1) at the lower part of the reactors, means (3) and (4) for the introduction and withdrawal of heat exchange fluid, characterized in that the tubular or plate like bundle is equipped with segmented partitions of the baffle plate, disc or crown type, irregularly distributed along the axis of the bundle, the distances separating to segmented partitions being minimal in the central part of the bundle along the reaction zone.

2. Reactor for nitration in the gaseous phase under pressure according to claim 1, characterized in that the reactor is equipped at the base with an allotment-distribution means among the different tubes of the bundle, provided with a sequence of enlargements and constrictions of the passage of the gases, especially a substantial constriction of the section of the passage along each tube of the bundle.

3. Reactor for nitration in the gaseous phase under pressure according to claim 2, characterized in that the allotment-distribution means comprises a zone of distribution of the gases (6) from where the conduits (7) emit, meeting distribution means (8) in communication with the distribution crowns ($8_1$, $8_2$, $8_3$), the said distribution means being in the axis of the tubes of the bundle (2) and at the base of the said, the same being provided with a calibrated passage (9) at their entrace and in the axis.

4. Reactor for nitration in the gaseous phase under pressure according to claim 1, comprising a reaction space containing a vertical tubular or plate like bundle the inner perimeter of the tubes of which is smaller than 800 mm, characterized in that the ratio between the surface of the bundle in contact with the reaction medium called available exchange surface, and the volume of the reaction space, S/V, is between 22 and 425 $m^{-1}$, preferably between 50 and 250 $m^{-1}$ and especially between 120 and 250 $m^{-1}$, and the thickness of the gaseous vein does not exceed 150 mm.

5. Reactor for nitration in a gaseous phase under pressure comprising a tubular bundle according to claim 1 and a heat exchange fluid of a suitable nature, characterized in that the distribution of the segmented partitions is such that the temperature of the outer skin of the tubes is maintained constantly near 25°C between the hottest point and the coldest point, and, moreover, the heat exchanges are such that the parameters — Q designating the heat yielded by the reaction, S the available exchange surface, U the global coefficient of heat exchange and Q' the total heat exchanged with the heat exchange fluid — Q/US is between 30 and 130, Q'/US is between 30 and 180 and U is between 12 and 200.

6. Reactor for nitration in the gaseous phase under pressure according to claim 2, characterized in that the residence time of the reaction gas mixture in the allotment-distribution means does not exceed 0.5 sec, preferably 0.1 sec, the velocities of the gas in the total apparatus being between 3 and 50 m/sec, the distribution of the throughput in the tubes of the bundle being such that the maximum difference of charge among the most charged tube and the least charged tube being atmost 3 percent.

7. Reactor for nitration according to claim 1, the bundle and allotment-distribution means of which constitute of corrosion resistant metals, characterized in that they are selected from the group of the inoxidable austenitic nickel-chromium steels.

Coupe A-A'

FIG.2

FIG.1

FIG.3

Coupe A-A'

FIG.4

Coupe B-B'

FIG.5

FIG. 6